# EUROPEAN PATENT APPLICATION

(11) **EP 3 574 858 A1**
(43) Date of publication of application: **04.12.2019**
(21) Application number: 19163007.8
(22) Date of filing: 14.03.2019
(51) Int. Cl.: A61B 18/00, A61B 18/12, A61B 18/14

(54) **CUTTING KNIFE WITH ADJUSTABLE NEEDLE AND USING METHOD THEREOF**

(30) Priority: 09.05.2018 CN 201810439021
(71) Applicant: Zhejiang Pengtian Medical Instrument Co., Ltd., Zhuji, Zhejiang Province 311800 (CN); Shen, Libao, Zhuji, Zhejiang Province 311800 (CN)
(72) Inventor: Huang, Zhigang, Zhuji, Zhejiang Province 311800 (CN); Si, Wangchun, Zhuji, Zhejiang Province 311800 (CN); Li, Leiyong, Zhuji, Zhejiang Province 311800 (CN)
(74) Representative: Loo, Chi Ching

(57) **Abstract**

The invention relates to the technical field of medical devices, in particular to a cutting knife with an adjustable needle and a using method thereof. An injection handle, a core rod, and a base have a first through hole, a second through hole, and a third through hole penetrating therethrough, respectively. One end of an injection tube is installed in the first through hole of the injection handle by slidably installing the injection handle in the second through hole of the core rod, and the injection tube passes through the second through hole and the third through hole. The other end is fixedly connected with the needle, the connecting tube is sleeved outside the injection tube and one end is connected to the core rod, and the other end is fixed with a cutter head. The needle slides in a fourth through hole of the cutter head. The using method includes the steps of electrocoagulation, injection and cutting, so that the extending and retraction movement of the needle in the cutter head can be controlled by controlling the injection handle, thereby adjusting the relative positions of the cutter head and the needle during the two processes of electrocoagulation and injection, greatly improving the convenience of operation. Further, the point stress of the needle can smoothly pierce the surface of tissue to perform injection and to reduce the risk of surgery.

## Description

### FIELD OF THE INVENTION

This invention relates to the technical field of medical instruments, in particular to a cutting knife with an adjustable needle and a using method thereof.

### BACKGROUND OF THE INVENTION

Cutting knife has been widely used in the medical field, especially in the use of microinvasive surgery due to the unique shape and small size of its cutter head. With function of electrocoagulation, the knife can be used to cut the tissue film precisely. Thus, the safety and stability of microinvasive surgery will be guaranteed.

The existing single-pole cutting knife mainly adopts a fixed connection mode of a cutter head and a needle, and the needle moving in the cutter head is not available. Further, because the cutter head needs to be electrocoagulated, the needle is usually mounted in the plane of the cutter head. Therefore, when the cutter head is pressed against the surface of the tissue for injection, it is necessary to press the cutter head hard to make the cutter head immersed inside the tissue, thereby providing the needle with a large injection pressure to pierce the tissue surface.

The above existing design of cutting knife has the following drawbacks: the needle cannot be moved relative to the cutter head, the relative positions of the cutter head and the needle cannot be adjusted during the two processes of electrocoagulation and injection, and the operation is inconvenient. This design of needle located in the plane of the cutter head cannot allow the needle to be inserted into the inside of tissue smoothly without pressing the needle hard. Press the cutter head hard to ensure that the needle pierces the surface of the tissue, easily causing damage to the bottom of the tissue. Further, it's difficult to master the pressing force, the requirement of user's proficiency is relatively high, and there is also a high risk of use.

### BRIEF SUMMARY OF THE INVENTION

This invention provides a cutting knife with an adjustable needle which solves the following drawbacks of the existing cutting knife.

A conventional needle cannot be moved relative to a cutter head, and the relative positions of the cutter head and the needle cannot be adjusted during the two processes of electrocoagulation and injection. The conventional operation is inconvenient. The conventional design of needle located in the plane of the cutter head cannot allow the needle to be inserted into the inside of tissue smoothly without pressing the needle hard. Press the cutter head hard to ensure that the needle pierces the surface of the tissue, easily causing damage to the bottom of the tissue. Further, it's difficult to master the pressing force, and the requirement of user's proficiency is relatively high, and there is also a high risk of surgery.

The invention also provides a method for using the cutting knife with the adjustable needle. According to the structural features of the cutting knife, the above technical problems can be solved by the using method.

The technical solutions are listed as followings.

A cutting knife with an adjustable needle, comprising an injection handle, a core rod, an electrode, a base, an injection tube, a connection tube, a needle, and a cutter head, wherein the injection handle has a first through hole running through an interior of the injection handle, the core rod has a second through hole running through an interior of the core rod, the base has a third through hole running through an interior of the base, the injection handle is slidably installed inside the second through hole, one end of the injection tube is installed inside the first through hole, the injection tube passes through the second through hole and the third through hole, the other end of the injection tube is fixedly connected with the needle, the core rod is installed at the base, the connection tube is sleeved outside the injection tube, one end of the connection tube is fixed inside the second through hole, the connection tube passes through the third through hole, the other end of the connection tube is fixedly connected with the cutter head, the cutter head has a fourth through hole running through an interior of the cutter head, the needle slides in the fourth through hole, the needle is capable of sliding out of the cutter head and comes out of a plane of the cutter head, the connection tube is made of a conductive material, the connection tube passes through the electrode and contacts the electrode, the electrode penetrates a sidewall of the base and is fixed on the base, the injection handle injects liquid into the injection tube through the first through hole and delivers the liquid to the needle through the injection tube, and the injection handle slides inside the second through hole to drive the injection tube to move, such that the needle is adjustable.

In a preferred embodiment, the core rod has a second slider, and the second slider is slidably installed inside the third through hole, an outer tube is fixed at a tail end of the third through hole of the base, the connection tube is sleeved inside the outer tube, and the connection tube is capable of being driven to move along an interior of the outer tube when the core rod slides along the third through hole.

In a preferred embodiment, an insulation sleeve is installed between an outer surface of the cutter head and an inner wall of the outer tube, the connection tube is connected with the cutter head through a limiting block, the limiting block is made of the conductive material, the limiting block slides along the inner wall of the outer tube, the cutter head extends out of the outer tube and drives the limiting block to move against the insulation sleeve, and the limiting block is used for limiting a length of the cutter head extending out of the outer tube.

In a preferred embodiment, one end of the cutter head extends out of the outer tube, a cutting end of the cutter head is located outside the outer tube, and a diameter of the cutting end is larger than a diameter of the outer tube to prevent the cutter head from retracting into the outer tube.

In a preferred embodiment, the connection tube is spring tube.

In a preferred embodiment, the core rod has a sliding slot and positioning holes, the injection handle has a first slider and a positioning bead, and the first slider is installed in the sliding slot, such that the injection handle is capable of sliding along an axis of the core rod, at least two positioning holes are provided along the axis of the core rod, and the positioning bead is engaged in the positioning hole such that the injection handle is fixed.

In a preferred embodiment, the injection tube is in fixed connection with the injection handle through a pushing tube.

In a preferred embodiment, a Luer connector is installed at a top of the injection handle, and the Luer connector communicates with the first through hole and is used to connect a syringe.

A using method of the cutting knife according to any of the above descriptions includes the following steps:
connecting the electrode with a high frequency device, and connecting a syringe with a Luer connector;
pushing the core rod, extending the cutter head, pulling out the injection handle, retracting the needle, turning on the high-frequency device, and performing electrocoagulation at tissue to be cut;
pulling out the core rod, retracting the cutter head, pushing the injection handle, extending the needle, pressing the cutter head against a middle of the tissue, immersing the needle into the tissue, and using the syringe for injection; and
pushing the core rod, extending the cutter head, pulling out the injection handle, retracting the needle, and cutting a raised portion after injection with the cutter head.

The beneficial effects of the invention are as follows.
(1) The injection handle are slidably installed into the second through hole of the core rod, one end of the injection tube is installed in the first through hole of the injection handle, the injection tube passes through the second through hole and the third through hole, the other end is in fixed connection with the needle, the connection sleeve is sleeved outside the injection tube and one end is connected to the core rod, the other end is fixed with a cutter head, and the needle can slide in the fourth through hole of the cutter head. Therefore, the telescopic movement of the needle in the cutter head can be controlled by controlling the injection handle, so that the relative positions of the cutter head and the needle can be adjusted during the two processes of electrocoagulation and injection, which improves the convenience of operation greatly.
(2) The needle is ensured to be able to slide out of the cutter head and come out of the plane of the cutter head, thereby forming a drop between the needle and the plane of the cutter head. The point stress of the needle can pierce the surface of tissue smoothly to perform injection when the cutter head is pressed against the surface of the tissue.
(3) Since it is not necessary to press the cutting knife hard, damage to the bottom of the tissue is avoided, which greatly reduces the requirement for users' proficiency and indirectly reducing the risk of surgery.

### BRIEF DESCRIPTION OF THE DRAWINGS

FIG. 1 is a perspective view of a cutting knife;
FIG. 2 is an exploded view of the cutting knife;
FIG. 3 is a cross-sectional view of the cutting knife when both of an injection handle and a core rod are pulled out;
FIG. 4 is a cross-sectional view of a cutter head when both of the injection handle and the core rod are pulled out;
FIG. 5 is a cross-sectional view of the cutting knife when both of the injection handle and the core rod are pushed in;
FIG. 6 is a cross-sectional view of the cutting knife when both of the injection handle and the core rod are pushed in;
FIG. 7 is a cross-sectional view of the cutter head in the steps of electrocoagulation and cutting during operation; and
FIG. 8 is a cross-sectional view of the cutter head in the injection step during operation.

The reference numerals in the figure are: 1: injection handle, 11: Luer connector, 12: first slider, 13: Positioning beads, 14: first through hole, 2: core rod, 21: sliding slot, 22: positioning hole, 23: second slider, 24: second through hole, 3: base, 31: electrode, 32: electrode sleeve, 33: upper base, 34: lower base, 35: third through hole, 4: booster tube, 5: injection tube, 6: spring tube, 7: outer tube, 71: insulation sleeve, 8: needle, 9: cutter head, and 91: limiting block.

### DETAILED DESCRIPTION OF THE INVENTION

This invention will be described in detail below with reference to appendix drawings.

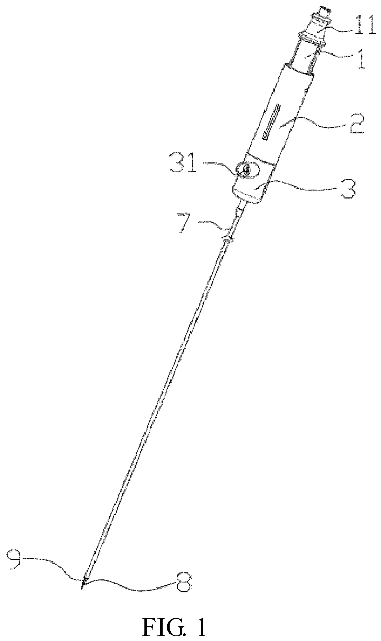

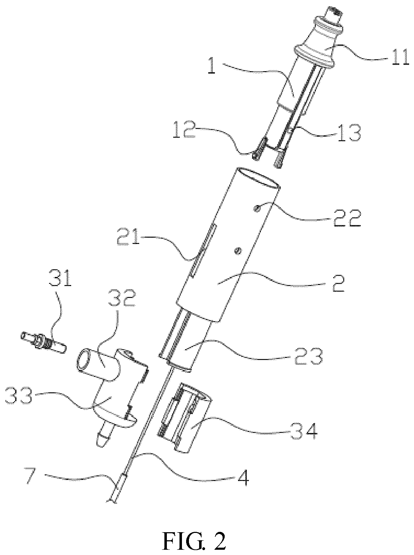

Please refer to FIG. 1 to FIG. 5. A cut knife in this invention includes an injection handle 1, a core rod 2, a base 3, an electrode 31, an injection tube 5, a spring tube 6, a needle 8, and a cutter head 9. A Luer connector 11 is installed fixedly at the top of the injection handle 1. The injection handle 1 has a first through hole 14 running through an interior of the injection handle 1, and an injection hole of the Luer connector 11 communicates with the first through hole 14 and is connected with a syringe (not shown in figures). Two first sliders 12 are disposed opposite to each other at the lower part of the injection handle 1, and two sides of the injection handle 1 are provided with one positioning bead 13, respectively. The opposite sides of the rod core 2 respectively have a sliding slot 21 connecting the outer surface and the inner wall thereof. Positioning holes 22 are respectively disposed at both sides of the sliding slot 21, and each group of the positioning holes 22 has two positioning holes 22. Insert the injection handle 1 into the second through hole 24 of the core rod 2, wherein the injection handle 1 slides along a bus direction of the core rod 2 is realized by the cooperation of the first sliders 12 and the sliding slots 21, and the positioning beads 13 can be snapped into the positioning holes 22, such that the injection handle 1 can control the locking of the needle in different gear positions. The lower part of the core rod 2 is provided with s a second slider 23. The base 3 is divided into an upper base 33, a lower base 34, and an electrode sleeve 32. The upper base 33 and the lower base 34 are fastened to each other, the inner wall thereof is provided with a third through hole 35, and the second slider 23 of the core rod 2 can slide in the second through hole 24, thereby controlling the stroke of cutter head 9. Pass the electrode 31 through the base 3 via the electrode sleeve 32, and make sure that the spring tube 6 passes through electrode 31 thus to conduct electricity via the spring tube 6, thereby completing electrocoagulation. An outer tube 7 is mounted below the base 33 for protecting the internal pipes and wires.

Hereinafter, different states of the cutting knife will be described in detail with reference to the cross-sectional views.

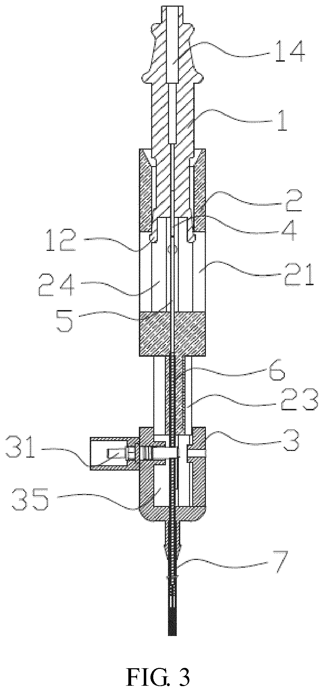

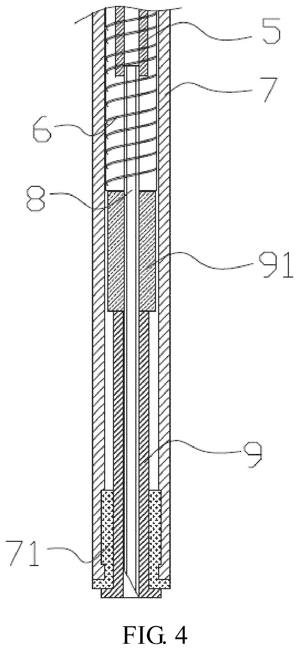

Please refer to FIG. 3 and FIG. 4. A booster tube 4 is fixed in the first through hole 14 of the injection handle 1, and one end of the booster tube 4 is connected to the injection tube 5. The injection tube 5 passes through the second through hole 24 of the core rod 2 and the third through hole 35 of the base 3 to be connected to the needle 8 at the other end. The spring tube 6 is mounted in the second through hole 24 of the core rod 2, and the spring tube 6 is sleeved outside the injection tube 5. Further, the spring tube 6 passes through the third through hole 35 and is connected to the cutter head 9 through the limiting block 91 at the other end. The spring tube 6 passes through the electrode 31 and is in contact with the electrode 31. The function of the spring tube 6 is to ensure that the cutter head 9 has a certain buffering effect in the process of contacting a surface of tissue, so as to avoid damage to the tissue caused by excessive force of the user. The outer tube 7 is fixed at the lower part of the base 3, and the outer tube 7 covers the spring tube 6 thus to provide protection. The spring tube 6, the cutter head 9, and the limiting block 91 are all made of stainless steel and have conductive properties .When a high frequency device is connected to the electrode 31, the current sequentially passes through the electrode 31, the spring tube 6, the limiting block 91, the cutter head 9, and the human body, such that a current loop is formed to achieve electrocoagulation. An insulation sleeve 71 is installed between one end of the outer tube 7 and the cutter head 9. The limiting block 91 is fixed on the top of the cutter head 9, and the functions of the limiting block 91 is that the limit block 91 is placed against the insulating block when the cutter head 9 is moved downward to prevent the cutter head 9 from completely coming out of the outer tube 7.

The injection handle 1 is located at the uppermost position, the first sliders 12 are against the top of the sliding slots 21, the positioning beads 13 are snapped into the upper positioning holes 22, and the injection handle 1 drives the injection tube 5 through the booster tube 4, thereby driving the needle 8 into the cutter head 9. At the same time, the core rod 2 is pulled out. At that time, the core rod 2 drives the spring tube 6 and the limiting block 91 to pull the cutter head 9 into retraction. The cross section of the cutter head 9 in the axial direction is in an inverted T shape, which ensures that the cutter head 9 can withstand the insulation sleeve 71 during the upward movement, thereby preventing the cutter head 9 from being completely retracted into the outer tube 7.

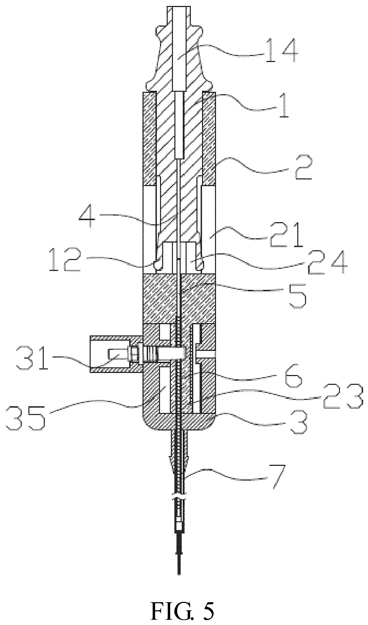

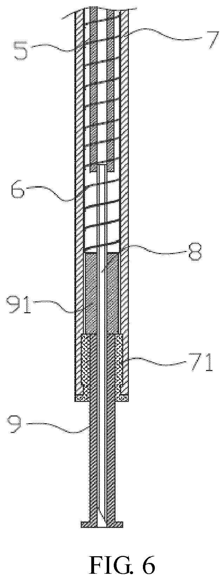

Please refer to FIG. 5 and FIG. 6. The injection handle 1 is located at the lowermost position, the first sliders 12 are against the bottom of the sliding slots 21, the positioning beads 13 are snapped into the lower positioning holes 22, and the injection handle 1 drives the injection tube 5 through the booster tube 4, thereby driving the needle 8 to extend out. At the same time, the core rod 2 is pushed in. At this time, the core rod 2 drives the spring tube 6 and the limiting block 91 to push the cutter head 9 out of the outer tube 7, and the limiting block 91 is against the insulation sleeve 71, thereby ensuring that the cutter head 9 will not come out of the outer tube 7.

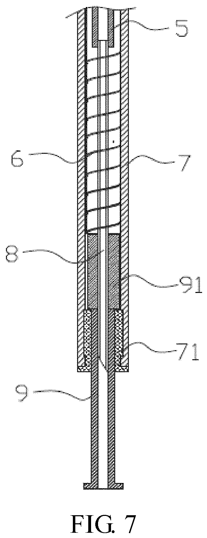

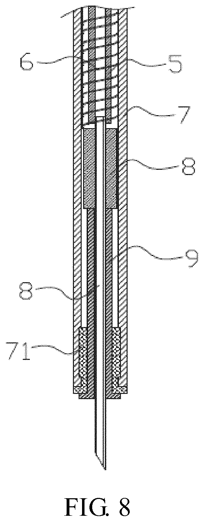

The method of using the above cutting knife includes the following steps:
(1) The electrode 31 a high frequency device, and the Luer connector 11 is connected to a syringe.
(2) As shown in FIG. 7, the high frequency device is turned on, the core rod 2 is pushed in, the cutter head 9 is extended out, and the limiting block 91 is pressed against the insulation sleeve 71; the injection handle 1 is pulled out, and the needle head 8 is retracted into the cutter head 9, and electrocoagulation is performed around the tissue to be cut thus to form a closed loop.
(3) As shown in FIG. 8, the injection handle 1 is pushed in to allow the needle 8 to extend out; the core rod 2 is pulled out, such that the cutter head 9 is retracted into the outer tube 7, and its contour in the inverted T shape is against the insulation sleeve 71. The cutter head 9 is placed against the surface of the tissue so that the needle 8 is completely immersed into the tissue, and the liquid to be injected is injected into the tissue to be cut by pushing the syringe, so that the uplift of the tissue can meet the requirement.
(4) Return the cutting knife to the state shown in FIG. 7, and cut the tissue.
(5) When cutting and requiring injection, the above steps (3) and (4) can be repeated to ensure that the tissue is completely separated from the human body.

## Claims

1. A cutting knife with an adjustable needle, comprising an injection handle, a core rod, an electrode, a base, an injection tube, a connection tube, a needle, and a cutter head, wherein the injection handle has a first through hole running through an interior of the injection handle, the core rod has a second through hole running through an interior of the core rod, the base has a third through hole running through an interior of the base, the injection handle is slidably installed inside the second through hole, one end of the injection tube is installed inside the first through hole, the injection tube passes through the second through hole and the third through hole, the other end of the injection tube is fixedly connected with the needle, the core rod is installed at the base, the connection tube is sleeved outside the injection tube, one end of the connection tube is fixed inside the second through hole, the connection tube passes through the third through hole, the other end of the connection tube is fixedly connected with the cutter head, the cutter head has a fourth through hole running through an interior of the cutter head, the needle slides in the fourth through hole, the needle is capable of sliding out of the cutter head and comes out of a plane of the cutter head, the connection tube is made of a conductive material, the connection tube passes through the electrode and contacts the electrode, the electrode penetrates a sidewall of the base and is fixed on the base, the injection handle injects liquid into the injection tube through the first through hole and delivers the liquid to the needle through the injection tube, and the injection handle slides inside the second through hole to drive the injection tube to move, such that the needle is adjustable.

2. A cutting knife with the adjustable needle according to claim 1, wherein the core rod has a second slider, and the second slider is slidably installed inside the third through hole, an outer tube is fixed at a tail end of the third through hole of the base, the connection tube is sleeved inside the outer tube, and the connection tube is capable of being driven to move along an interior of the outer tube when the core rod slides along the third through hole.

3. A cutting knife with the adjustable needle according to claim 2, wherein an insulation sleeve is installed between an outer surface of the cutter head and an inner wall of the outer tube, the connection tube is connected with the cutter head through a limiting block, the limiting block is made of the conductive material, the limiting block slides along the inner wall of the outer tube, the cutter head extends out of the outer tube and drives the limiting block to move against the insulation sleeve, and the limiting block is used for limiting a length of the cutter head extending out of the outer tube.

4. A cutting knife with the adjustable needle according to claim 3, wherein one end of the cutter head extends out of the outer tube, a cutting end of the cutter head is located outside the outer tube, and a diameter of the cutting end is larger than a diameter of the outer tube to prevent the cutter head from retracting into the outer tube.

5. A cutting knife with the adjustable needle according to any one of claims 1-4, wherein the connection tube is a spring tube.

6. A cutting knife with the adjustable needle according to any one of claims 1-4, wherein the core rod has a sliding slot and positioning holes, the injection handle has a first slider and a positioning bead, and the first slider is installed in the sliding slot, such that the injection handle is capable of sliding along an axis of the core rod, at least two positioning holes are provided along the axis of the core rod, and the positioning bead is engaged in the positioning hole such that the injection handle is fixed.

7. A cutting knife with the adjustable needle according to any one of claims 1-4, wherein the injection tube is in fixed connection with the injection handle through a pushing tube.

8. A cutting knife with the adjustable needle according to any one of claims 1-4, wherein a Luer connector is installed at a top of the injection handle, and the Luer connector communicates with the first through hole and is used to connect a syringe.

9. A method for using a cutting knife according to any one of claims 1-8, comprising the following steps:
connecting the electrode with a high frequency device, and connecting a syringe with a Luer connector;
pushing the core rod, extending the cutter head, pulling out the injection handle, retracting the needle, turning on the high-frequency device, and performing electrocoagulation at tissue to be cut;
pulling out the core rod, retracting the cutter head, pushing the injection handle, extending the needle, pressing the cutter head against a middle of the tissue, immersing the needle into the tissue, and using the syringe for injection; and
pushing the core rod, extending the cutter head, pulling out the injection handle, retracting the needle, and cutting a raised portion after injection with the cutter head.
